# EUROPÄISCHE PATENTANMELDUNG

(11) **EP 1 250 939 A1**
(43) Veröffentlichungstag der Anmeldung: **23.10.2002**
(21) Anmeldenummer: 02005260.1
(22) Anmeldetag: 11.03.2002
(51) Int. Cl.: A61L 9/02, A61K 7/46

(54) **Temperaturstabile Polymere als Träger für Duftstoffe**

(30) Priorität: 23.03.2001 DE 10114517
(71) Anmelder: HAARMANN & REIMER GMBH, 37603 Holzminden (DE)
(72) Erfinder: Mansfeld, Gerd, 37632 Eschershausen (DE); Harzke, Falk, 37619 Bodenwerder (DE); Eilers, Jörg, 37619 Hehlen (DE); Bork, Karl-Hein, 37627 Deensen (DE)
(74) Vertreter: Mann, Volker, Dr.

(57) **Zusammenfassung**

Die Erfindung betrifft die Verwendung von temperaturstabilen Polyimid-haltigen Polymeren als Träger von Duftstoffen, ein Verfahren zur thermischen Duftstoffausbringung sowie temperaturstabile Polymere enthaltend Polyimide und Duftstoffe.

## Beschreibung

Die Erfindung betrifft die Verwendung von temperaturstabilen Polyimid-haltigen Polymeren als Träger von Duftstoffen, ein Verfahren zur thermischen Duftstoffausbringung sowie temperaturstabile Polymere enthaltend Polyimide und Duftstoffe.

Es bestand die Aufgabe, ein Wärmeübertragungssystem zu entwickeln, mit dem Duftstoffe oder Parfümkompositionen in sehr kurzer Zeit auf Temperaturen bis zu 300°C erwärmt werden können.

Aufgrund der Tatsache, dass Duftstoffe oder Parfümkompositionen teilweise mit Metallen reagieren (z.B. Farbreaktionen), musste ein temperatur-, hitze- und parfümbeständiger Werkstoff gefunden werden, der die Wärme sehr gut leitet. Für diesen Zweck wurden die in der Elektroindustrie verwendeten Wärmeleitfolien mit unterschiedlichen Materialeigenschaften geprüft.

Ein Gegenstand der vorliegenden Erfindung ist die Verwendung von temperaturstabilen Polyimid-haltigen Polymeren als Träger oder Speicher für die thermische Ausbringung von Duftstoffen.

Ein weiterer Gegenstand der vorliegenden Erfindung ist ein Verfahren zur thermischen Duftstoffausbringung, dadurch gekennzeichnet, dass der Duftstoffträger ein temperaturstabiles Polyimid-haltiges Polymer enthält.

Weiterhin ist ein Gegenstand der vorliegenden Erfindung ein temperaturstabiles Polyimid-haltiges Polymer enthaltend Duftstoffe.

Temperaturstabile Polymere im Sinne der Erfindung sind Polymere mit einem Schmelzpunkt oberhalb von 140°C, insbesondere oberhalb von 200°C.

Erfindungsgemäß besonders geeignet sind temperaturstabile Polymere, die einen Polyimid-Anteil von größer 50 Gew.-%, bevorzugt von größer 70 Gew.-% und besonders bevorzugt von größer 85 Gew.-% aufweisen. Vorteilhaft sind Polyimide aus Pyromellitsäureanhydrid und Diaminen, bevorzugt aromatischen Diaminen. Die erfindungsgemäßen Polymere können beispielsweise Polymere wie poly- oder perfluorierte Kohlenwasserstoffe (z.B. Teflon®) enthalten.

In einer bevorzugten Ausführungsform werden die temperaturstabilen Polymere als Folie oder Film verwendet. Die Filmdicke ist unkritisch. Typischerweise liegen die Filmdicken im Bereich von 10 bis 250 µm, bevorzugt im Bereich von 25 bis 150 µm.

Polyimide sind, auch als Polyimid-haltige Filme, beispielsweise unter dem Markennamen Kapton® (Markenname der Firma DuPont) im Markt erhältlich.

Als besonders vorteilhafte Ausführungsformen der Polyimid-haltigen Polymere seien beispielsweise genannt: Typ Kapton® HN (Polymer aus Pyromellitsäureanhydrid und 4,4-Diaminodiphenylether, Typ Kapton® FN (eine Kombination aus Kapton® HN und Teflon®).

In einer weiteren bevorzugten Ausführungsform liegen die temperaturstabilen Polymere als getrennte Hohlraum-Speichereinheiten vor, in denen die Duftstoffe separat oder gemeinsam erwärmt werden können.

Die erfindungsgemäßen Duftstoffe können flüssig, hochviskos, pastös, gelartig, glasartig, fest, verkapselt, eingebettet, umhüllt, granuliert, verschmolzen oder eingeschmolzen vorliegen. Die Duftstoffe können Einzelriechstoffe, Riechstoffmischungen, (komplexe) Parfümkompositionen oder Parfümöle sein. In einer bevorzugten Ausführungsform werden verkapselte Duftstoffe verwendet. Die Duftstoffe können in, auf oder umgeben von dem temperaturstabilen Polymer sein.

Die Erwärmung der Duftstoffe oder deren Ausbringung erfolgt bevorzugt bei Temperaturen oberhalb von 100°C, besonders bevorzugt im Bereich von 120 bis 300°C und ganz besonders bevorzugt im Bereich von 180 bis 250°C.

Die erfindungsgemäßen Polymere wurden auf ihre thermische Stabilität und ihre Parfümöl-Beständigkeit geprüft.

### Prüfung auf Parfümöl-Beständigkeit:

Die Duftstoffe und Parfümöl-Beständigkeit der Wärmeleitfolien wurden mit den 100 meistgebrauchten Parfümeinsatzstoffen und mit 30 Parfümölen geprüft.

Zu dieser Prüfung wurden auf die Wärmeleitfolien etwa 10 µl der zu prüfenden Flüssigkeiten aufgegeben. Die Beurteilung auf Beständigkeit wurde nach 1 h, 1 Tag und 10 Tagen visuell durchgeführt. Dabei wurde insbesondere auf Verformung, Verfärbung und Anlöserscheinungen geachtet.

### Prüfung auf thermische Stabilität:

Zur Prüfung auf thermische Stabilität wurde eine beheizbare temperaturgesteuerte Aluminium-Heizfläche verwendet, auf die die zu testende Wärmeleitfolie aufgespannt war.

Zu dieser Prüfung wurden 10 µl der unter Punkt 1 aufgeführten Duftstoffe und komplexe Parfümöle auf die Wärmeleitfolie (Abmessungen: 2 x 3 cm; Dicke: 102 µm) gegeben, und die Temperatur der Aluminium-Heizfläche langsam (5°C/min) von 120°C auf die vom Hersteller angegebene Maximaltemperatur gesteigert. Die Beurteilung wurde geruchlich und visuell durchgeführt. Bei der geruchlichen Prüfung wurde auf geruchliche Veränderungen der Parfümeinsatzstoffe und der Parfümöle geachtet sowie auf "Fremdgerüche" die eventuell durch die Wärmeleitfolie verursacht wurden. Bei der visuellen Beurteilung lag das Augenmerk insbesondere auf Verformung, Verfärbung und Anlöseerscheinungen.

Bei diesen Versuchen wurde festgestellt, dass Wärmeleitfolien aus Silicon-Verbindungen den Ansprüchen nicht entsprechen.

Wärmeleitfolien mit Kontaktklebstoffen (einseitig oder zweiseitig) fielen durch starke "Fremdgerüche" bei Wärmebelastung auf.

Gute Resultate wurden mit Wärmeleitfolien erzielt, die in Sandwich-Bauweise aufgebaut waren und deren Kontaktflächen aus Polyimidfolie bestand.

## Patentansprüche

1. Verwendung von temperaturstabilen Polyimid-haltigen Polymeren als Träger oder Speicher von Duftstoffen.

2. Verwendung von temperaturstabilen Polyimid-haltigen Polymeren zur thermischen Ausbringung von Duftstoffen.

3. Verwendung nach Anspruch 2, **dadurch gekennzeichnet, dass** die Ausbringung bei einer Temperatur oberhalb von 100°C durchgeführt wird.

4. Verwendung nach wenigstens einem der Ansprüche 1 bis 3, **dadurch gekennzeichnet, dass** der Polyimid-Gehalt im temperaturstabilen Polymer größer als 50 Gew.-% beträgt.

5. Verwendung nach wenigstens einem der Ansprüche 1 bis 4, **dadurch gekennzeichnet, dass** die Duftstoffe verkapselt sind.

6. Verwendung nach wenigstens einem der Ansprüche 1 bis 5, **dadurch gekennzeichnet, dass** das temperaturstabile Polymer als Folie ausgebildet ist.

7. Verfahren zur thermischen Duftstoffausbringung, **dadurch gekennzeichnet, dass** der Duftstoffträger ein temperaturstabiles Polyimid-haltiges Polymer enthält.

8. Verfahren nach Anspruch 7, **dadurch gekennzeichnet, dass** der Duftstoffträger auf eine Temperatur oberhalb von 100°C erwärmt wird

9. Temperaturstabiles Polyimid-haltiges Polymer enthaltend Duftstoffe.

10. Polymer nach Anspruch 9, **dadurch gekennzeichnet, dass** der Polyimid-Gehalt größer als 50 Gew.-% ist.
